# EUROPÄISCHE PATENTANMELDUNG

(11) **EP 4 671 754 A1**
(43) Veröffentlichungstag der Anmeldung: **31.12.2025**
(21) Anmeldenummer: 25183514.6
(22) Anmeldetag: 18.06.2025
(51) Int. Cl.: G01N 33/00

(54) **VORRICHTUNG ZUR ALTERNIERENDEN ZUFÜHRUNG EINES NULLGASES UND EINES MESSGASES ZU EINEM GASSENSOR UND VERFAHREN ZUR BESTIMMUNG EINER KONZENTRATION EINES ZIELGASES IN EINEM MESSGAS**

(30) Priorität: 25.06.2024 DE 102024117846
(71) Anmelder: Dräger Safety AG & Co. KGaA, 23560 Lübeck (DE)
(72) Erfinder: Nauber, Andreas, 23558 Lübeck (DE); Haupt, Stephan, 23558 Lübeck (DE); Traving, Wulf-Gerd, 23558 Lübeck (DE); Beckmann, Udo, 23558 Lübeck (DE); Meyer, Benjamin, 23558 Lübeck (DE); Will, Folke, 23558 Lübeck (DE)

(57) **Zusammenfassung**

Vorrichtung zur alternierenden Zuführung eines Nullgases und eines Messgases zu einem Gassensor. Die Vorrichtung weist eine erste Gaszuführung zur Zuführung eines Nullgases und eine zweite Gaszuführung zur Zuführung eines Messgases auf, wobei die zweite Gaszuführung von der ersten Gaszuführung verschieden ist. Die Vorrichtung weist ferner eine Sensoraufnahme zur Aufnahme eines Gassensors sowie Mittel zum Beeinflussen von Gasfluss in der Vorrichtung auf, welche eingerichtet sind, einen Fluss von Messgas durch die erste Gaszuführung zu der Sensoraufnahme und einen Fluss von Nullgas durch die zweite Gaszuführung zu der Sensoraufnahme bereitzustellen. Die Vorrichtung weist ferner eine Steuereinheit auf, welche eingerichtet ist, die Mittel derart zu steuern, dass der Fluss von Messgas und der Fluss von Nullgas alternierend zu der Sensoraufnahme geleitet werden, wobei die erste Gaszuführung mit einer Gasabführung verbindbar oder verbunden ist, wobei die Gasabführung von der ersten Gaszuführung und von der zweiten Gaszuführung verschieden ist, und wobei die Steuereinheit ferner eingerichtet ist, die Mittel derart zu steuern, dass während (d.h. zumindest zeitanteilig) der Fluss von Messgas zu der Sensoraufnahme geleitet wird, der Fluss von Nullgas durch die Gasabführung in eine Umgebung der Vorrichtung geleitet wird. Es wird ferner ein entsprechendes Verfahren bereitgestellt.

## Beschreibung

Die vorliegende Erfindung betrifft eine Vorrichtung zur alternierenden Zuführung eines Nullgases und eines Messgases zu einem Gassensor. Die Erfindung betrifft ferner ein Verfahren zur Bestimmung einer Konzentration eines Zielgases in einem Messgas.

Verwandte Vorrichtungen sind beispielsweise aus DE 10 159 616 B4 und aus US 10 509 007 B1 bekannt.

Es ist eine Aufgabe der vorliegenden Erfindung, eine alternative Vorrichtung und ein entsprechendes Verfahren bereitzustellen.

Erfindungsgemäß wird insofern eine Vorrichtung zur alternierenden Zuführung eines Nullgases und eines Messgases zu einem Gassensor, insbesondere zur Bestimmung einer Konzentration eines Zielgases in dem Messgas, bereitgestellt. Die Vorrichtung weist eine erste Gaszuführung zur Zuführung eines Nullgases und eine zweite Gaszuführung zur Zuführung eines Messgases auf, wobei die zweite Gaszuführung von der ersten Gaszuführung verschieden ist. Die Vorrichtung weist ferner eine Sensoraufnahme zur Aufnahme eines Gassensors sowie Mittel zum Beeinflussen von Gasfluss in der Vorrichtung auf, welche eingerichtet sind, einen Fluss von Messgas durch die erste Gaszuführung zu der Sensoraufnahme und einen Fluss von Nullgas durch die zweite Gaszuführung zu der Sensoraufnahme bereitzustellen. Die Vorrichtung weist ferner eine Steuereinheit auf, welche eingerichtet ist, die Mittel derart zu steuern, dass der Fluss von Messgas und der Fluss von Nullgas alternierend zu der Sensoraufnahme geleitet werden, wobei die erste Gaszuführung mit einer Gasabführung verbindbar oder verbunden ist, wobei die Gasabführung von der ersten Gaszuführung und von der zweiten Gaszuführung verschieden ist, und wobei die Steuereinheit ferner eingerichtet ist, die Mittel derart zu steuern, dass während (d.h. zumindest zeitanteilig) der Fluss von Messgas zu der Sensoraufnahme geleitet wird, der Fluss von Nullgas durch die Gasabführung in eine Umgebung der Vorrichtung geleitet wird.

Auf diese Weise kann eine gattungsgemäße Vorrichtung bereitgestellt werden, welche eine abwechselnde Zuführung eines Nullgases und eines Messgases zu der Sensoraufnahme bzw. zu dem Gassensor ermöglicht und somit ein abwechselndes Nullen des Gassensors mittels des Nullgases und anschließendes Messen eines Zielgases in dem Messgas zur Verbesserung des Messverhaltens des Gassensors ermöglicht.

Die Erfindung beruht dabei auf der Erkenntnis, dass ein Umleiten des Flusses von Nullgas durch die Gasabführung während der Zuführung des Flusses von Messgas zum Gassensor ein verbessertes Anströmen des Gassensors zur Folge hat, da so keine Strömungsunterbrechung oder Strömungsrichtungswechsel entlang des Gassensors erforderlich sind. Auf diese Weise kann das Messverhalten des Gassensors vorteilhaft verbessert werden.

Insbesondere ist es mit der erfindungsgemäßen Vorrichtung möglich, in einem ersten Betriebszustand der Vorrichtung ein stabiles Nullsignal des Gassensors durch Begasung mit Nullgas zu ermitteln und in einem zweiten Betriebszustand der Vorrichtung eine Bestimmung der Konzentration von Zielgas im Messgas Störeinflüsse durch Berücksichtigung des Nullsignals zu kompensieren. Unter Verwendung von rauscharmen Gassensoren - beispielsweise rauscharmer elektrochemischer Gassensoren - und vorzugsweise periodisch wiederholender Nullstrommessung können so Zielgaskonzentrationen im Bereich von ppb (parts per billion) gemessen werden. Mit der erfindungsgemäßen Vorrichtung kann jedoch auch die Empfindlichkeit von Halbleitersensoren und anderen Sensorsystemen verbessert werden. Mit der erfindungsgemäßen Vorrichtung lassen sich beispielsweise die Gase NH3, NO2, PH3, COCl2 und H2S im einstelligen ppb-Bereich messen.

Die erfindungsgemäße Vorrichtung kann vorzugsweise als Modul bereitgestellt und mit einem Gassensor verbunden werden.

Jede Gaszuführung und jede Gasabführung kann als eine oder mehrere Gasleitungen ausgestaltet sein oder eine oder mehrere Gasleitungen umfassen. Jede Gasleitung kann beispielsweise als Kanal oder als Schlauch ausgestaltet sein.

Unter einem Messgas wird ein Gas oder Gasgemisch verstanden, welches ein Zielgas, also eine Komponente, deren Konzentration in dem Messgas untersucht werden soll, umfassen kann. Das Messgas kann beispielsweise aus der Umgebung der Vorrichtung erhältlich sein.

Unter einem Nullgas wird ein Gas oder Gasgemisch verstanden, welches zur Nullung, Kalibrierung und/oder Bestimmung eines Referenzwertes für den Gassensor geeignet ist. In einem bevorzugten Fall werden sowohl das Messgas als auch das Nullgas aus einer Umgebung der Vorrichtung entnommen und das Nullgas durch wenigstens teilweise, vorzugsweise vollständige Entfernung des Zielgases aus dem Umgebungsgas erhalten. Das entnommene Umgebungsgas ohne wenigstens teilweise entferntes Zielgases, also das Nullgas samt Zielgas, kann in diesem Fall als Messgas fungieren.

Dieses Vorgehen eignet sich besonders, wenn nur geringe Konzentrationen von Zielgas in dem Umgebungsgas vorliegen bzw. potentiell vorliegen und das Umgebungsgas veränderliche Eigenschaften aufweisen kann, welche als Störeinflüsse auf die Messung des Zielgases wirken können. Durch Zuführung des Umgebungsgases mit verringertem Zielgas-Anteil oder im Wesentlichen ohne Zielgas als Nullgas kann somit eine Nullmessung (Nullung, Kalibrierung, Bestimmung eines Referenzwertes) in Bezug auf das Umgebungsgas durchgeführt werden. Eine zeitlich nachgelagerte Messung mit Messgas ermöglicht in diesem Fall eine von Störeinflüssen wenigstens teilweise befreite Aussage über die Konzentration des Zielgases in dem Messgas. Beispielsweise kann die Konzentration des Zielgases in dem Messgas proportional zu einer Differenz des Gassensor-Signals der Messung mit Messgas und dem Gassensor-Signal der Nullmessung sein.

Es ist möglich, dass das Nullgas durch eine von der Quelle des Messgases verschiedene Quelle bereitgestellt wird, beispielsweise durch eine Gasflasche, welche ein Nullgas mit vorbestimmten Eigenschaften bereitstellen kann.

Die Steuereinheit kann signal- und/oder energietechnisch mit den Mitteln zum Beeinflussen von Gasfluss in der Vorrichtung verbindbar oder verbunden sein. Die Steuereinheit kann ferner eingerichtet sein, Messsignale des Gassensors zu empfangen, auszuwerten und optional auszugeben, beispielsweise mittels einer Anzeigeeinheit wie einem Display oder mittels einer datentechnischen Schnittstelle, beispielsweise mittels einer kabellosen oder kabelgebunden Schnittstelle.

Die Steuereinheit kann ganz oder teilweise als Hardwareschaltung implementiert sein, welche beispielsweise Gate-Arrays, handelsübliche Halbleiter wie Logikchips, Transistoren oder andere diskrete Komponenten umfassen kann. Die Steuereinheit kann ebenso in programmierbaren Hardware-Bauteilen wie feldprogrammierbaren Gate-Arrays, programmierbarer Array-Logik, programmierbaren Logikbauteilen oder Ähnlichem implementiert sein. Die Steuereinheit kann ebenso in Software für die Ausführung durch verschiedene Arten von Prozessoren implementiert sein und zum Beispiel ein oder mehrere physische oder logische Module von Computeranweisungen umfassen, die zum Beispiel als Objekt, Prozedur oder Funktion organisiert sein können. Die Steuereinheit kann beispielsweise als Computer, Prozessor, Mikroprozessor, (feld-) programmierbarer Logik-Array ((F)PLAs = (Field) Programmable Logic Array), (feld-)programmierbare Gate-Array ((F)PGA = (Field) Programmable Gate Array), Digitalsignalprozessor-Hardware (DSP-Hardware; DSP = Digital Signal Processor), anwendungsspezifische integrierte Schaltung (ASIC = Application Specific Integrated Circuit), und/oder feldprogrammierbare Logikanordnung (FPGA = Field Programmable Gate Array) ausgestaltet sein.

Unter einer alternierenden Leitung des Flusses von Messgas und des Flusses von Nullgas zu der Sensoraufnahme wird verstanden, dass die genannten Flüsse, vorzugsweise periodisch wiederholend, abwechselnd zu der Sensoraufnahme geleitet werden.

Die Gasabführung ist vorzugsweise eine von einer ersten Gasleitung der ersten Gaszuführung und von einer zweiten Gasleitung der zweiten Gaszuführung verschiedene (dritte) Gasleitung mit einem Auslass, welcher von den Einlass der ersten Gasleitung und von einem Auslass der zweiten Gasleitung verschieden ist.

Der Gassensor kann beispielsweise als elektrochemischer Gassensor oder als Halbleitersensor ausgestaltet sein.

Vorzugsweise umfassen die Mittel zum Beeinflussen von Gasfluss in der Vorrichtung eine erste Gasfördereinrichtung, eine zweite Gasfördereinrichtung oder ein Mehrwegeventil, und eine dritte Gasfördereinrichtung, wobei die erste Gasfördereinrichtung eingerichtet ist, den Fluss von Nullgas zu der Sensoraufnahme zu bewirken, wobei die dritte Gasfördereinrichtung eingerichtet ist, den Fluss von Messgas zu der Sensoraufnahme zu bewirken, und wobei die zweite Gasfördereinrichtung oder das Mehrwegeventil eingerichtet ist, dass während der Fluss von Messgas zu der Sensoraufnahme geleitet wird, der Fluss von Nullgas durch die Gasabführung in die Umgebung der Vorrichtung geleitet wird.

Auf diese Weise ist eine besonders einfache Implementierung der erfindungsgemäßen Vorrichtung möglich.

Unter einer Gasfördereinrichtung wird ein Bauelement oder eine Baugruppe zum Transport, also zum Fördern und/oder Bewegen, von Gas verstanden. Beispiele von Gasfördereinrichtungen sind Pumpen, Gebläse (auch bezeichnet als Ventilatoren) und/oder Verdichter.

Bevorzugt ist das Mehrwegeventil als ein 3/2-Wegeventil ausgestaltet.

Vorzugsweise ist die dritte Gasfördereinrichtung ferner ausgebildet, den Fluss von Nullgas zu der Sensoraufnahme zu bewirken.

Auf diese Weise kann eine Funktionsintegration erreicht werden.

Vorzugsweise münden die erste Gaszuführung und die zweite Gaszuführung strömungsaufwärts von der Sensoraufnahme in einem ersten Mischpunkt in eine gemeinsame Zuführleitung, wobei die erste Gasfördereinrichtung strömungsaufwärts von dem ersten Mischpunkt angeordnet ist, wobei die dritte Gasfördereinrichtung strömungsabwärts von den ersten Mischpunkt angeordnet ist, wobei die dritte Gasleitung in einem strömungsaufwärts des Mischpunkts liegenden Bereich von der ersten Gaszuführung abzweigt, und wobei die zweite Gasfördereinrichtung oder das Mehrwegeventil in oder an der Gasabführung angeordnet ist.

Auf diese Weise ist eine besonders einfache Implementierung der erfindungsgemäßen Vorrichtung möglich.

Ein Mischpunkt bezeichnet einen Bereich, welcher für eine Zusammenführung und/oder Mischung von Gasen geeignet ist. Ein Mischpunkt kann beispielsweise durch ein T-Stück oder durch eine Mischkammer ausgestaltet sein.

Bevorzugt zweigt die dritte Gasleitung ferner in einem strömungsabwärts der ersten Gasfördereinrichtung liegenden Bereich ab. Dies ist aber nicht erforderlich, die erste Gasfördereinrichtung kann auch strömungsabwärts von der Gasabführung angeordnet sein, solange sie strömungsaufwärts von dem ersten Mischpunkt angeordnet ist.

Vorzugsweise weisen die erste Gasfördereinrichtung, die zweite Gasfördereinrichtung, wenn vorhanden, und die dritte Gasfördereinrichtung jeweils einen piezoelektrischen Aktor auf.

Ein Beispiel einer solchen Gasfördereinrichtung wird als Mikropumpe bezeichnet und ist aus JP 2009 074 418 A2 und aus US 2009 010 779 A bekannt. Hierzu kann die Gasfördereinrichtung eine Pumpenkammer aufweisen, welche eine Schwingmembran aufweist, wobei ein piezoelektrischer auf einer Seite der Schwingmembran angeordnet ist, wobei die Schwingmembran durch eine Betätigung, beispielsweise Biegedeformation, des piezoelektrischen Aktors deformierbar ist und das Volumen der Pumpenkammer verändert wird, um Fluid durch die Pumpenkammer zu befördern.

Vorzugsweise ist eine Flussrate der ersten Gasfördereinrichtung im eingeschalteten Zustand größer als eine Flussrate der dritten Gasfördereinrichtung im eingeschalteten Zustand.

Somit kann der Fluss des Nullgases vorteilhaft zu der Sensoraufnahme geleitet und gleichzeitig in der Nähe von der Sensoraufnahme befindliches Messgas verdrängt werden.

Vorzugsweist ist eine Flussrate der zweiten Gasfördereinrichtung im eingeschalteten Zustand größer als die Flussrate der dritten Gasfördereinrichtung im eingeschalteten Zustand, und die Flussrate der zweiten Gasfördereinrichtung im eingeschalteten Zustand größer als die Flussrate der ersten Gasfördereinrichtung im eingeschalteten Zustand.

Somit kann der Fluss des Messgases vorteilhaft zu der Sensoraufnahme geleitet und gleichzeitig in der Nähe von der Sensoraufnahme befindliches Nullgas verdrängt werden.

Bevorzugt entspricht die Flussrate der zweiten Gasfördervorrichtung im Wesentlichen der Summe aus der Flussrate der ersten Gasfördervorrichtung und der der Flussrate der dritten Gasfördervorrichtung.

Vorzugsweise sind die erste Gasfördereinrichtung und die zweite Gasfördereinrichtung oder das Mehrwegeventil, und die dritte Gasfördereinrichtung zusammen mit der ersten Gaszuführung, der zweiten Gaszuführung und der Gasabführung in einem gemeinsamen Gehäuse aufgenommen oder ausgebildet.

Auf diese Weise kann eine kompakt ausgebildete Vorrichtung bereitgestellt werden.

Vorzugsweise weist die Vorrichtung ferner einen Fluss-Sensor und/oder einen Druck-Sensor, welcher strömungstechnisch mit der zweiten Gaszuführung verbunden ist, auf, wobei die Steuereinheit ferner eingerichtet ist: Messsignale des Fluss-Sensors und/oder des Druck-Sensors zu erhalten, aus den Messsignalen einen Strömungszustand in der zweiten Gaszuführung zu bestimmen, den bestimmten Strömungszustand mit einem vorbestimmten Strömungszustand zu vergleichen, welcher einer ordnungsgemäßen Funktion der Mittel entspricht, aus dem Vergleich zu bestimmen, ob die Mittel ordnungsgemäß funktionieren, und eine entsprechende Ausgabe bereitzustellen.

Somit kann auf einfache Weise eine Überprüfung der ordnungsgemäßen Funktion der Mittel zum Beeinflussen von Gasfluss in der Vorrichtung ermöglicht werden.

Der vorbestimmte Strömungszustand kann als vorbestimmte Information vorliegen, welche den vorbestimmte Strömungszustand indiziert. Die vorbestimmte Information kann der Steuereinheit mittels einer Speichereinheit bereitgestellt werden. Die Speichereinheit kann beispielsweise als Komponente der Vorrichtung vorliegen und beispielsweise als ein Direktzugriffsspeicher, ein Cache-Speicher, ein Nur-Lese-Speicher (ROM), ein löschbarer programmierbarer Nur-Lese-Speicher (EPROM) und ein elektrisch löschbarer programmierbarer Nur-Lese-Speicher (EEPROM) und/oder als ein Flash-Speicher ausgestaltet sein.

Vorzugsweise ist der Fluss-Sensor und/oder der Druck-Sensor in der zweiten Gaszuführung angeordnet.

Vorzugsweise ist in der ersten Gaszuführung ein Element zur wenigstens teilweisen Entfernung eines Zielgases aus dem durch das Element führbaren Gas angeordnet, oder vorzugsweise ist die erste Gaszuführung mit einem Element zur wenigstens teilweisen Entfernung eines Zielgases aus dem durch das Element führbaren Gas verbunden.

Beispiele für Elemente zur wenigstens teilweisen Entfernung eines Zielgases aus dem durch das Element führbaren Gas sind Gaswäscher (Scrubber), Filter und Pyrolyseelemente.

Vorzugsweise weist die Vorrichtung ferner ein Heizelement zur Beheizung wenigstens eines Teils der Vorrichtung, insbesondere der Gaszuführungen und/oder der Sensoraufnahme auf.

Ein Kondensationsrisiko innerhalb der Vorrichtung kann somit vorteilhaft verringert werden.

Das Heizelement kann beispielsweise als Heizfolie und/oder als Heizdraht ausgestaltet sein. Weitere Bauformen von Heizelementen sind bekannt und möglich.

Vorzugsweise weist die Vorrichtung ferner einen stromaufwärts von der Sensoraufnahme angeordneten Feuchtepuffer auf.

Schwankungen der Feuchte des durch den Feuchtepuffer geleiteten Gases können somit vorteilhaft verringert werden.

Der Feuchtepuffer kann beispielsweise als Flüssigkeitsbehältnis ausgestaltet sein, wobei das eingeleitete Gas über die Flüssigkeitsoberfläche oder durch die Flüssigkeit hindurch geleitet werden kann. Die Flüssigkeit kann beispielsweise als Salzlösung ausgestaltet sein. Der Feuchtepuffer kann ferner beispielsweise als poröser Festkörper ausgestaltet sein, durch den oder an dem das Gas geleitet werden kann, um die Feuchte des Gases durch Feuchteaufnahme (Absorption) und/oder Feuchteabgabe (Desorption) zu beeinflussen. Weitere Bauformen von Feuchtepuffern sind bekannt und möglich.

Erfindungsgemäß wird ferner ein Verfahren zur Bestimmung einer Konzentration eines Zielgases in einem Messgas bereitgestellt. Das Verfahren weist die folgenden Verfahrensschritte auf: Bereitstellen eines Flusses von Nullgas durch eine erste Gaszuführung zu einem Gassensor in einem ersten Zeitabschnitt, Bereitstellen eines Flusses von Messgas durch eine zweite Gaszuführung zu dem Gassensor in einem zweiten Zeitabschnitt, welcher von dem ersten Zeitabschnitt verschieden ist, und Bereitstellen des Flusses von Nullgas durch eine Gasabführung während des zweiten Zeitabschnitts (d.h. zumindest zeitweise während des zweiten Zeitabschnitts, vorzugsweise während mehr als 50 % des zweiten Zeitabschnitts, besonders vorzugsweise während mehr als 90 % des zweiten Zeitabschnitts, ganz besonders bevorzugt während des vollen zweiten Zeitabschnitts), wobei die erste Gaszuführung mit der Gasabführung und diese mit einer Umgebung der Vorrichtung verbunden ist.

Das erfindungsgemäße Verfahren stellt zu der erfindungsgemäßen Vorrichtung vergleichbare Vorteile und Wirkungen bereit. Alle in Bezug auf die Vorrichtung beschriebenen Merkmale gelten als im Zusammenhang mit dem Verfahren offenbart und umgekehrt. Das Verfahren kann insofern insbesondere mittels der erfindungsgemäßen Vorrichtung durchgeführt werden. Dabei kann die Steuereinheit einige oder alle Schritte des Verfahrens ausführen oder deren Ausführung veranlassen. Hierzu kann eine maschinen-, prozessor- oder computerlesbare Programmspeichervorrichtung entsprechende Programme von Anweisungen codieren, so dass die Anweisungen einige oder alle der Schritte der oben beschriebenen Verfahren ausführen oder deren Ausführung veranlassen. Die Programmspeichervorrichtung kann z. B. als Digitalspeicher ausgestaltet sein.

Weitere Merkmale der Erfindung ergeben sich aus der nachfolgenden Figurenbeschreibung. Dabei zeigt:
- **Fig. 1a**: eine schematische Darstellung eines ersten Ausführungsbeispiels einer erfindungsgemäßen Vorrichtung in einem ersten Betriebszustand,
- **Fig. 1b**: eine schematische Darstellung des ersten Ausführungsbeispiels der erfindungsgemäßen Vorrichtung in einem zweiten Betriebszustand,
- **Fig. 2a**: eine schematische Darstellung eines zweiten Ausführungsbeispiels einer erfindungsgemäßen Vorrichtung in einem ersten Betriebszustand,
- **Fig. 2b**: eine schematische Darstellung des zweiten Ausführungsbeispiels der erfindungsgemäßen Vorrichtung in einem zweiten Betriebszustand,
- **Fig. 3**: im oberen Bereich ein schematisches Messsignal-Zeit-Diagramm und im unteren Bereich ein schematisches Flussraten-Zeit-Diagramm,
- **Fig. 4**: ein schematischer Ablaufplan des erfindungsgemäßen Verfahrens, und
- **Fig. 5**: eine schematische Darstellung einer erfindungsgemäßen Gasfördereinrichtung mit piezoelektrischem Aktor.

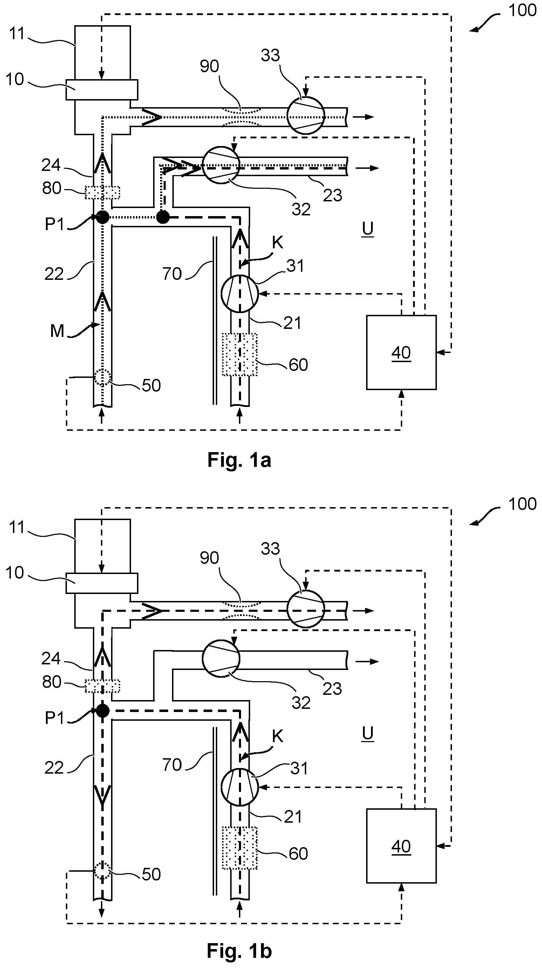

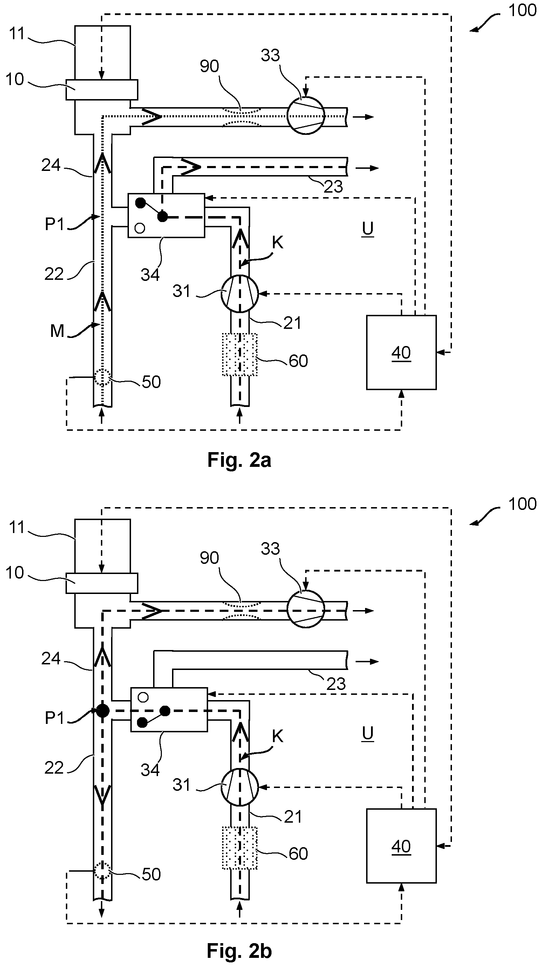

Erfindungsgemäß wird eine Vorrichtung 100 zur alternierenden Zuführung eines Nullgases K und eines Messgases M zu einem Gassensor 10 bereitgestellt. Ein erstes Ausführungsbeispiel einer solchen Vorrichtung ist in Fig. 1a und 1b dargestellt. Ein zweites Ausführungsbeispiel einer solchen Vorrichtung ist in Fig. 2a und 2b dargestellt. Sofern im Folgenden lediglich von einer oder der Vorrichtung 100 gesprochen wird, sind alle möglichen Ausführungsbeispiele der erfindungsgemäßen Vorrichtung 100 - gezeigt oder nicht gezeigt - gemeint.

Die Vorrichtung 100 weist, wie dies in Fig. 1a, 1b, 2a und 2b dargestellt ist, eine erste Gaszuführung 21 zur Zuführung eines Nullgases K und eine zweite Gaszuführung 22 zur Zuführung eines Messgases M auf, wobei die zweite Gaszuführung 22 von der ersten Gaszuführung 21 verschieden ist. Die Vorrichtung 100 weist ferner eine Sensoraufnahme 11 zur Aufnahme eines Gassensors 10 auf. Die Vorrichtung 100 weist ferner Mittel 31, 32, 33, 34 zum Beeinflussen von Gasfluss in der Vorrichtung 100 auf, welche eingerichtet sind, einen Fluss von Messgas M durch die erste Gaszuführung 22 zu der Sensoraufnahme 11 und einen Fluss von Nullgas K durch die zweite Gaszuführung 21 zu der Sensoraufnahme 11 bereitzustellen. Die Vorrichtung 100 weist ferner eine Steuereinheit 40 auf, welche eingerichtet ist, die Mittel 31, 32, 33, 34 derart zu steuern, dass der Fluss von Messgas M und der Fluss von Nullgas K alternierend zu der Sensoraufnahme 11 geleitet werden, wobei die erste Gaszuführung 21 mit einer Gasabführung 23 verbindbar oder verbunden ist, wobei die Gasabführung 23 von der ersten Gaszuführung 21 und von der zweiten Gaszuführung 23 verschieden ist, und wobei die Steuereinheit 40 ferner eingerichtet ist, die Mittel 31, 32, 33, 34 derart zu steuern, dass während der Fluss von Messgas M zu der Sensoraufnahme 11 geleitet wird, der Fluss von Nullgas K durch die Gasabführung 23 in eine Umgebung U der Vorrichtung 100 geleitet wird.

Es ist in allen Ausführungsformen möglich, dass die Steuereinheit 40 in signaltechnischer Verbindung mit den Mitteln 31, 32, 33, 34 und/oder mit dem Gassensor 10 und/oder der Sensoraufnahme 11 steht.

In dem ersten Ausführungsbeispiel nach Fig. 1a, 1b umfassen die Mittel 31, 32, 33 eine erste Gasfördereinrichtung 31, eine zweite Gasfördereinrichtung 32 und eine dritte Gasfördereinrichtung 33. Die erste Gasfördereinrichtung 31 ist eingerichtet, den Fluss von Nullgas K zu der Sensoraufnahme 11 zu bewirken, wobei die dritte Gasfördereinrichtung 33 eingerichtet ist, den Fluss von Messgas M zu der Sensoraufnahme 11 zu bewirken, und wobei die zweite Gasfördereinrichtung 32 eingerichtet ist, dass während der Fluss von Messgas M zu der Sensoraufnahme 11 geleitet wird, der Fluss von Nullgas K durch die Gasabführung 23 in die Umgebung der Vorrichtung 100 geleitet wird.

In dem zweiten Ausführungsbeispiel nach Fig. 2a, 2b umfassen die Mittel 31, 33, 34 eine erste Gasfördereinrichtung 31, ein Mehrwegeventil 34 und eine dritte Gasfördereinrichtung 33. Die erste Gasfördereinrichtung 31 ist eingerichtet, den Fluss von Nullgas K zu der Sensoraufnahme 11 zu bewirken, wobei die dritte Gasfördereinrichtung 33 eingerichtet ist, den Fluss von Messgas M zu der Sensoraufnahme 11 zu bewirken, und wobei das Mehrwegeventil 34 eingerichtet ist, dass während der Fluss von Messgas M zu der Sensoraufnahme 11 geleitet wird, der Fluss von Nullgas K durch die Gasabführung 23 in die Umgebung der Vorrichtung 100 geleitet wird.

In jedem Ausführungsbeispiel kann die dritte Gasfördereinrichtung 33 ausgebildet sein, den Fluss von Nullgas K zu der Sensoraufnahme 11 zu bewirken.

In jedem Ausführungsbeispiel können die erste Gaszuführung 21 und die zweite Gaszuführung 22 strömungsaufwärts von der Sensoraufnahme 11 in einem ersten Mischpunkt P1 in eine gemeinsame Zuführleitung 24 münden, wobei die erste Gasfördereinrichtung 31 strömungsaufwärts von dem ersten Mischpunkt P1 angeordnet sein kann, wobei die dritte Gasfördereinrichtung 33 strömungsabwärts von den ersten Mischpunkt P1 angeordnet sein kann, wobei die dritte Gasleitung 23 in einem strömungsaufwärts des Mischpunkts P1 liegenden Bereich von der ersten Gaszuführung 21 abzweigen kann, und wobei die zweite Gasfördereinrichtung 32 oder das Mehrwegeventil 34 in oder an der Gasabführung 23 angeordnet sein kann.

Es ist in jeder Ausführungsform bevorzugt, dass die erste Gasfördereinrichtung 31, die zweite Gasfördereinrichtung 32, wenn vorhanden, und die dritte Gasfördereinrichtung 33 jeweils einen piezoelektrischen Aktor 31a aufweist.

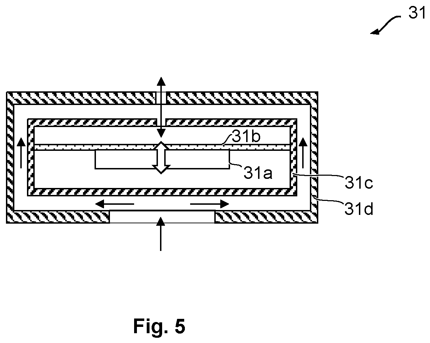

Ein Beispiel einer Gasfördereinrichtung 31 mit einem piezoelektrische Aktor 31a ist in Fig. 5 dargestellt. Jede der Gasfördereinrichtungen 31, 32, 33 kann vergleichbar ausgestaltet sein. In dem Beispiel nach Fig. 5 ist in einem äußeren Gehäuse 31d ein inneres Gehäuse 31c, beabstandet zu dem äußeren Gehäuse 31d, zur Bildung von Strömungspfaden angeordnet, welches über eine ausgangsseitige Öffnung strömungstechnisch mit den Strömungspfaden zwischen äußerem Gehäuse 31d und innerem Gehäuse 31c verbunden ist. In dem inneren Gehäuse 31c ist eine Schwingmembran 31b gelagert, an welcher der piezoelektrische Aktor 31a angeordnet ist. Durch Betätigung des piezoelektrische Aktors 31a kann dieser in (z.B. Biege-)Schwingungen versetzt werden, wodurch die Schwingmembran 31b zu einer Formveränderung veranlasst wird (angedeutet durch den Doppelpfeil in Fig. 5). In Folge der Formveränderung wird das Volumen zwischen äußerem Gehäuse 31d und Schwingmembran 31b verändert und Gas aus den Strömungspfaden eingesaugt bzw. ausgestoßen. Dies passiert in schneller Abfolge, typischerweise im Ultraschall-Bereich, so dass die Gasfördereinrichtung 31 auf diese Weise in kompakter Bauform ausreichend hohe, quasi-kontinuierliche Flussraten bereitstellen kann. Es ergibt sich so ein Fluss (Volumenstrom) entlang der schematisch dargestellten schwarzen Pfeile.

Es ist in jeder Ausführungsform bevorzugt, dass jede Gasfördereinrichtung 31, 32, 33 durch eine (betriebspunktabhängige) Flussrate F im eingeschalteten Zustand gekennzeichnet sein kann. Insofern ist ferner bevorzugt, dass eine Flussrate F1 der ersten Gasfördereinrichtung 31 im eingeschalteten Zustand größer ist als eine Flussrate F3 der dritten Gasfördereinrichtung 33 im eingeschalteten Zustand. Es ist ferner bevorzugt, dass eine Flussrate F2 der zweiten Gasfördereinrichtung 32 im eingeschalteten Zustand größer ist als die Flussrate F3 der dritten Gasfördereinrichtung 33 im eingeschalteten Zustand, und dass die Flussrate F2 der zweiten Gasfördereinrichtung 32 im eingeschalteten Zustand größer ist als die Flussrate F1 der ersten Gasfördereinrichtung 31 im eingeschalteten Zustand.

Es wird nun eine bevorzugte Funktion der Vorrichtung 100 nach Fig. 1a, 1b beschrieben.

In einem ersten Betriebszustand, welcher in Fig. 1a dargestellt ist und in welchem das Messgas M zu der Sensoraufnahme 11 mit Gassensor 10 gelangen soll, sind alle drei Gasfördereinrichtungen 31, 32 und 33 aktiv. Die erste Gasfördereinrichtung 31 fördert Gas mit der Flussrate F1 aus der Umgebung U und leitet dieses als Nullgas K in Richtung der Sensoraufnahme 11, wie durch den Gaspfad mit langer Strichelung angedeutet ist. Die dritte Gasfördereinrichtung 33, welche strömungsabwärts von der Sensoraufnahme 11 angeordnet ist, fördert Gas aus der Umgebung U als Messgas M in Richtung der Sensoraufnahme 11 und am Gassensor 10 vorbei, wie durch den Gaspfad mit kurzer Strichelung angedeutet ist. Da die Flussrate F2 der zweiten Gasfördereinrichtung 32 größer ist als die Flussrate F1 der ersten Gasfördereinrichtung 31 und als die Flussrate F3 der dritten Gasfördereinrichtung 33, fördert die zweite Gasfördereinrichtung 32 einen Teil des Messgases M und das gesamte Nullgas K durch die Gasabführung 23 in die Umgebung U, wie durch die überlagerten Gaspfade mit langer Strichelung und mit kurzer Strichelung angedeutet ist, so dass das Nullgas K nicht zur Sensoraufnahme 11 gelangt und die Messung des Messgases M nicht verfälschen kann. Gleichzeitig ist so gewährleistet, dass die Förderung des Nullgases K nicht unterbrochen werden muss, was bei einem Wechsel von dem ersten Betriebszustand in den zweiten Betriebszustand vorteilhaft ist.

In einem zweiten Betriebszustand, welcher in Fig. 1b dargestellt ist und in welchem das Nullgas K zu der Sensoraufnahme 11 mit Gassensor 10 gelangen soll, sind lediglich die erste Gasfördereinrichtung 31 und die dritte Gasfördereinrichtung 33 aktiv. Da die Flussrate F1 der ersten Gasfördereinrichtung 31 höher ist als die Flussrate F3 der dritten Gasfördereinrichtung 33, gelangt ein Teil des Nullgases K zu der Sensoraufnahme 11 und zu Gassensor 10, während ein anderer Teil entlang der ersten Gaszuführung 21 und durch die zweite Gaszuführung 22 in Richtung der Umgebung U gefördert wird. Somit kann ein unerwünschter Einfluss von Messgas M auf den Gassensor 10 im zweiten Betriebszustand verhindert bzw. verringert werden.

Es ist ersichtlich, dass bei einem Wechsel von dem ersten Betriebszustand in den zweiten Betriebszustand und andersherum lediglich die zweite Gasfördereinrichtung 32 in oder an der Gasabführung 23 (de)aktiviert werden muss und die übrigen Gasfördereinrichtungen 31, 33 aktiv sein können. Das Abreißen von Strömungen entlang des Gassensors 10 bzw. durch die Sensoraufnahme 11 und entsprechende turbulente Effekte können so wirksam verhindert bzw. abgeschwächt werden, was hohe Umschaltfrequenzen zwischen den Betriebszuständen und folglich ein verbessertes Messverhalten ermöglicht.

Die Funktion der Vorrichtung 100 nach Fig. 2a, 2b entspricht der Funktion der Vorrichtung 100 nach Fig. 1a, 1b, wobei jedoch der Fluss von Nullgas K im ersten Betriebszustand (Fig. 2a) nicht durch Fördern mittels einer dritten Gasfördereinrichtung 33 bewerkstelligt wird, sondern durch Umschalten des Strömungspfades des Nullgases K mittels des Mehrwegeventils 34. Im ersten Betriebszustand (Fig. 2a) verbindet das Mehrwegeventil 34 insofern die erste Gaszuführung 21 mit der Gasabführung 23, so dass nur das Messgas M zu der Sensoraufnahme 11 und Gassensor 10 gelangen kann, wie durch den Gaspfad mit kurzer Strichelung angedeutet ist. In dem zweiten Betriebszustand (Fig. 2b) verbindet das Mehrwegeventil 34 die erste Gaszuführung 21 mit der gemeinsamen Zuführleitung 24, so dass nur das Nullgas K zu der Sensoraufnahme 11 und Gassensor 10 gelangen kann, wie durch den Gaspfad mit langer Strichelung angedeutet ist.

Es ist in jeder Ausführungsform möglich, dass stromaufwärts von der dritten Gasfördereinrichtung 33 eine Drossel 90 angeordnet ist, wie dies schematisch in Fig. 1a, 1b, 2a, 2b angedeutet ist.

Es ist in jeder Ausführungsform möglich, dass die erste Gasfördereinrichtung 31 und die zweite Gasfördereinrichtung 32 oder das Mehrwegeventil 34, und die dritte Gasfördereinrichtung 33 zusammen mit der ersten Gaszuführung 21, der zweiten Gaszuführung 22 und der Gasabführung 23 in einem gemeinsamen Gehäuse aufgenommen oder ausgebildet sind.

Die Vorrichtung 100 kann in jeder Ausführungsform einen Fluss-Sensor 50 und/oder einen Druck-Sensor aufweisen, welcher strömungstechnisch mit der zweiten Gaszuführung 22 verbunden sein kann. Dies ist in Fig. 1a, 1b, 2a, 2b dargestellt. Die Steuereinheit 40 kann ferner eingerichtet sein: Messsignale S des Fluss-Sensors 50 und/oder des Druck-Sensors zu erhalten, aus den Messsignalen S einen Strömungszustand in der zweiten Gaszuführung 22 zu bestimmen, den bestimmten Strömungszustand mit einem vorbestimmten Strömungszustand zu vergleichen, welcher einer ordnungsgemäßen Funktion der Mittel 31, 32, 33, 34 entspricht, aus dem Vergleich zu bestimmen, ob die Mittel 31, 32, 33, 34 ordnungsgemäß funktionieren, und eine entsprechende Ausgabe bereitzustellen. Dabei kann der Fluss-Sensor 50 und/oder der Druck-Sensor in der zweiten Gaszuführung 22 angeordnet sein.

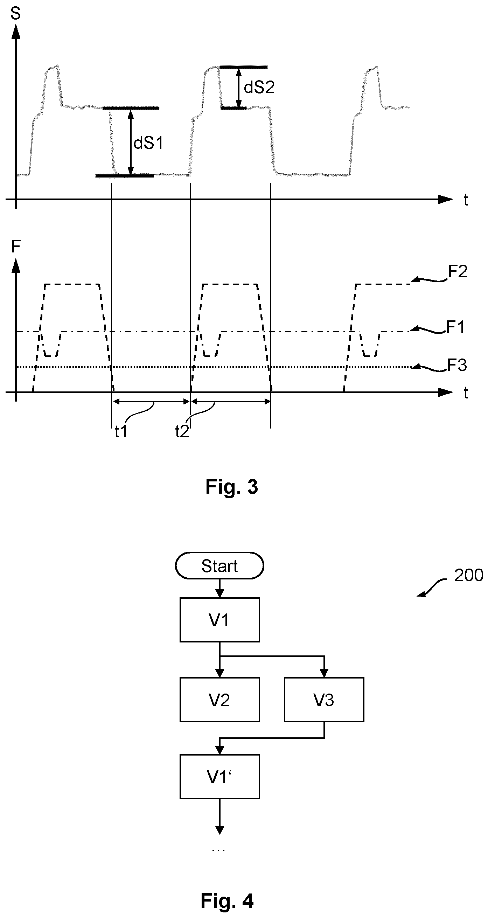

Fig. 3 zeigt im oberen Bereich ein Messsignal S- Zeit t -Diagramm, welches mit einem solchen Fluss-Sensor 50 und/oder der Druck-Sensor erhältlich ist. Fig. 3 zeigt im unteren Bereich ein korrespondierendes schematisches Flussraten F-Zeit t -Diagramm. Durch Auswertung des Messsignal S- Zeit t-Verlaufs können relevante Kenngrößen abgeleitet werden. Aus Fig. 3 ist insofern ersichtlich, dass in einem Beispiel einer Steuerung der Vorrichtung 100 des ersten Ausführungsbeispiels die dritte Gasfördereinrichtung 33 konstant mit der Flussrate F3 betrieben werden kann. Die zweite Gasfördereinrichtung 32 kann zur Bereitstellung des ersten Betriebszustands, beispielsweise wie gezeigt periodisch, für eine Zeitdauer t2 mit der Flussrate F2 aktiviert und zur Bereitstellung des zweiten Betriebszustands für eine Zeitdauer t1 deaktiviert werden. Der Unterschied im entsprechenden Messsignal S zwischen eingeschalteter dritter Gasfördereinrichtung 33 und nicht eingeschalteter dritter Gasfördereinrichtung 33 als Signaldifferenz dS1 kann so bestimmt und mit einer entsprechenden vorbestimmten Signaldifferenz verglichen werden, um zu ermitteln, ob eine ordnungsgemäße Funktion vorliegt. Die erste Gasfördervorrichtung 31 kann ebenfalls konstant mit der Flussrate F1 betrieben werden oder, wie gezeigt, mit kurzzeitiger Anpassung der Flussrate F1 betrieben werden. Dies ermöglicht ebenfalls eine Auswertung des Unterschieds im entsprechenden Messsignal S zwischen der Bereitstellung der normalen Flussrate F1 und der angepassten Flussrate F1 als Signaldifferenz dS2. Diese Signaldifferenz dS2 kann ebenso mit einer entsprechenden vorbestimmten Signaldifferenz verglichen werden, um zu ermitteln, ob eine ordnungsgemäße Funktion vorliegt.

Es ist in allen Ausführungsbeispielen möglich, dass in der ersten Gaszuführung 21 ein Element 60 zur wenigstens teilweisen Entfernung eines Zielgases aus dem durch das Element 60 führbaren Gas angeordnet ist, oder dass die erste Gaszuführung 21 mit einem Element 60 zur wenigstens teilweisen Entfernung eines Zielgases aus dem durch das Element 60 führbaren Gas verbunden ist. Dies ist in Fig. 1a, 1b, 2a, 2b dargestellt.

Es ist in allen Ausführungsbeispielen möglich, dass die Vorrichtung 100 ferner ein Heizelement 70 zur Beheizung wenigstens eines Teils der Vorrichtung 100 aufweist. Dies ist jedoch lediglich in Fig. 1a, 1b dargestellt. Dort ist das Heizelement 70 lediglich entlang der ersten Gaszuführung 21 angeordnet, es kann aber ebenso in einem anderen oder allen Bereichen der Vorrichtung 100 angeordnet sein.

Es ist in allen Ausführungsbeispielen möglich, dass die Vorrichtung 100 ferner einen stromaufwärts von der Sensoraufnahme 11 angeordneten Feuchtepuffer 80 aufweist. Dies ist jedoch lediglich in Fig. 1a, 1b dargestellt.

In Fig. 4 ist ein Beispiel eines erfindungsgemäßen Verfahrens 200 zur Bestimmung einer Konzentration eines Zielgases in einem Messgas M dargestellt. Das Verfahren 200 weist die Verfahrensschritte auf:
V1: Bereitstellen eines Flusses von Nullgas K durch eine erste Gaszuführung 21 zu einem Gassensor 10 in einem ersten Zeitabschnitt t1.
V2: Bereitstellen eines Flusses von Messgas M durch eine zweite Gaszuführung 22 zu dem Gassensor 10 in einem zweiten Zeitabschnitt t2, welcher von dem ersten Zeitabschnitt t1 verschieden ist.
V3: Bereitstellen des Flusses von Nullgas K durch eine Gasabführung 23 während des zweiten Zeitabschnitts t2, wobei die erste Gaszuführung 21 mit der Gasabführung 23 und diese mit einer Umgebung U der Vorrichtung 100 verbunden ist.

Es ist möglich, dass das Verfahren 200 periodisch wiederholend durchgeführt wird.

Alle hierin offenbarten Merkmale sind beliebig miteinander kombinierbar, sofern dies nicht Alternativen betrifft oder widersprüchlich ist.
- 10: **Bezugszeichenliste** Gassensor
- 11: Sensoraufnahme
- 21: Erste Gaszuführung
- 22: Zweite Gaszuführung
- 23: Gasabführung
- 24: Zuführleitung
- 31: Mittel zum Beeinflussen von Gasfluss in der Vorrichtung, erste Gasfördereinrichtung
- 31a: piezoelektrischer Aktor
- 31b: Schwingmembran
- 31c: inneres Gehäuse
- 31d: äußeres Gehäuse
- 32: Mittel zum Beeinflussen von Gasfluss in der Vorrichtung, zweite Gasfördereinrichtung
- 33: Mittel zum Beeinflussen von Gasfluss in der Vorrichtung, dritte Gasfördereinrichtung
- 34: Mittel zum Beeinflussen von Gasfluss in der Vorrichtung, Mehrwegeventil
- 40: Steuereinheit
- 50: Fluss-Sensor
- 60: Element zur wenigstens teilweisen Entfernung eines Zielgases
- 70: Heizelement
- 80: Feuchtepuffer
- 90: Drossel
- 100: Vorrichtung
- 200: Verfahren

- dS1: erster Signalunterschied
- dS2: zweiter Signalunterschied
- F: Flussrate
- F1: Flussrate der ersten Gasfördereinrichtung
- F2: Flussrate der zweiten Gasfördereinrichtung
- F3: Flussrate der dritten Gasfördereinrichtung
- K: Nullgas
- M: Messgas
- P1: erster Mischpunkt
- S: Messsignale
- t: Zeit
- t1: erster Zeitabschnitt
- t2: zweiter Zeitabschnitt
- U: Umgebung der Vorrichtung
- V1, V2,: Schritte des Verfahrens

## Patentansprüche

1. Vorrichtung (100) zur alternierenden Zuführung eines Nullgases (K) und eines Messgases (M) zu einem Gassensor (10), aufweisend:
- eine erste Gaszuführung (21) zur Zuführung eines Nullgases (K),
- eine zweite Gaszuführung (22) zur Zuführung eines Messgases (M), wobei die zweite Gaszuführung (22) von der ersten Gaszuführung (21) verschieden ist,
- eine Sensoraufnahme (11) zur Aufnahme eines Gassensors (10),
- Mittel (31, 32, 33, 34) zum Beeinflussen von Gasfluss in der Vorrichtung (100), welche eingerichtet sind, einen Fluss von Messgas (M) durch die erste Gaszuführung (22) zu der Sensoraufnahme (11) und einen Fluss von Nullgas (K) durch die zweite Gaszuführung (21) zu der Sensoraufnahme (11) bereitzustellen, und
- eine Steuereinheit (40), welche eingerichtet ist, die Mittel (31, 32, 33, 34) derart zu steuern, dass der Fluss von Messgas (M) und der Fluss von Nullgas (K) alternierend zu der Sensoraufnahme (11) geleitet werden,
wobei die erste Gaszuführung (21) mit einer Gasabführung (23) verbindbar oder verbunden ist, wobei die Gasabführung (23) von der ersten Gaszuführung (21) und von der zweiten Gaszuführung (23) verschieden ist, und
wobei die Steuereinheit (40) ferner eingerichtet ist, die Mittel (31, 32, 33, 34) derart zu steuern, dass während der Fluss von Messgas (M) zu der Sensoraufnahme (11) geleitet wird, der Fluss von Nullgas (K) durch die Gasabführung (23) in eine Umgebung (U) der Vorrichtung (100) geleitet wird.

2. Vorrichtung (100) nach Anspruch 1,
wobei die Mittel (31, 32, 33, 34) umfassen:
- eine erste Gasfördereinrichtung (31),
- eine zweite Gasfördereinrichtung (32) oder ein Mehrwegeventil (34), und
- eine dritte Gasfördereinrichtung (33),
wobei die erste Gasfördereinrichtung (31) eingerichtet ist, den Fluss von Nullgas (K) zu der Sensoraufnahme (11) zu bewirken,
wobei die dritte Gasfördereinrichtung (33) eingerichtet ist, den Fluss von Messgas (M) zu der Sensoraufnahme (11) zu bewirken, und
wobei die zweite Gasfördereinrichtung (32) oder das Mehrwegeventil (34) eingerichtet ist, dass während der Fluss von Messgas (M) zu der Sensoraufnahme (11) geleitet wird, der Fluss von Nullgas (K) durch die Gasabführung (23) in die Umgebung der Vorrichtung (100) geleitet wird.

3. Vorrichtung (100) nach Anspruch 2,
wobei die dritte Gasfördereinrichtung (33) ferner ausgebildet ist, den Fluss von Nullgas (K) zu der Sensoraufnahme (11) zu bewirken.

4. Vorrichtung (100) nach Anspruch 2 oder 3,
wobei die erste Gaszuführung (21) und die zweite Gaszuführung (22) strömungsaufwärts von der Sensoraufnahme (11) in einem ersten Mischpunkt (P1) in eine gemeinsame Zuführleitung (24) münden,
wobei die erste Gasfördereinrichtung (31) strömungsaufwärts von dem ersten Mischpunkt (P1) angeordnet ist,
wobei die dritte Gasfördereinrichtung (33) strömungsabwärts von den ersten Mischpunkt (P1) angeordnet ist,
wobei die dritte Gasleitung (23) in einem strömungsaufwärts des Mischpunkts (P1) liegenden Bereich von der ersten Gaszuführung (21) abzweigt, und
wobei die zweite Gasfördereinrichtung (32) oder das Mehrwegeventil (34) in oder an der Gasabführung (23) angeordnet ist.

5. Vorrichtung (100) nach einem der Ansprüche 2 bis 4,
wobei die erste Gasfördereinrichtung (31), die zweite Gasfördereinrichtung (32), wenn vorhanden, und die dritte Gasfördereinrichtung (33) jeweils einen piezoelektrischen Aktor (31a) aufweisen.

6. Vorrichtung (100) nach einem der Ansprüche 2 bis 5,
wobei eine Flussrate (F1) der ersten Gasfördereinrichtung (31) im eingeschalteten Zustand größer ist als eine Flussrate (F3) der dritten Gasfördereinrichtung (33) im eingeschalteten Zustand.

7. Vorrichtung (100) nach Anspruch 6,
wobei eine Flussrate (F2) der zweiten Gasfördereinrichtung (32) im eingeschalteten Zustand größer ist als die Flussrate (F3) der dritten Gasfördereinrichtung (33) im eingeschalteten Zustand, und
wobei die Flussrate (F2) der zweiten Gasfördereinrichtung (32) im eingeschalteten Zustand größer ist als die Flussrate (F1) der ersten Gasfördereinrichtung (31) im eingeschalteten Zustand.

8. Vorrichtung (100) nach einem der Ansprüche 2 bis 7,
wobei die erste Gasfördereinrichtung (31) und die zweite Gasfördereinrichtung (32) oder das Mehrwegeventil (34), und die dritte Gasfördereinrichtung (33) zusammen mit der ersten Gaszuführung (21), der zweiten Gaszuführung (22) und der Gasabführung (23) in einem gemeinsamen Gehäuse (...) aufgenommen oder ausgebildet sind.

9. Vorrichtung (100) nach einem der Ansprüche 1 bis 8,
ferner aufweisend einen Fluss-Sensor (50) und/oder einen Druck-Sensor, welcher strömungstechnisch mit der zweiten Gaszuführung (22) verbunden ist,
wobei die Steuereinheit (40) ferner eingerichtet ist:
- Messsignale (S) des Fluss-Sensors (50) und/oder des Druck-Sensors zu erhalten,
- aus den Messsignalen (S) einen Strömungszustand in der zweiten Gaszuführung (22) zu bestimmen,
- den bestimmten Strömungszustand mit einem vorbestimmten Strömungszustand zu vergleichen, welcher einer ordnungsgemäßen Funktion der Mittel (31, 32, 33, 34) entspricht,
- aus dem Vergleich zu bestimmen, ob die Mittel (31, 32, 33, 34) ordnungsgemäß funktionieren, und
- eine entsprechende Ausgabe bereitzustellen.

10. Vorrichtung (100) nach Anspruch 9,
wobei der Fluss-Sensor (50) und/oder der Druck-Sensor in der zweiten Gaszuführung (22) angeordnet ist.

11. Vorrichtung (100) nach einem der vorherigen Ansprüche,
wobei in der ersten Gaszuführung (21) ein Element (60) zur wenigstens teilweisen Entfernung eines Zielgases aus dem durch das Element (60) führbaren Gas angeordnet ist, oder
wobei die erste Gaszuführung (21) mit einem Element (60) zur wenigstens teilweisen Entfernung eines Zielgases aus dem durch das Element (60) führbaren Gas verbunden ist.

12. Vorrichtung (100) nach einem der vorherigen Ansprüche,
ferner aufweisend ein Heizelement (70) zur Beheizung wenigstens eines Teils der Vorrichtung (100).

13. Vorrichtung (100) nach einem der vorherigen Ansprüche,
ferner aufweisend einen stromaufwärts von der Sensoraufnahme (11) angeordneten Feuchtepuffer (80).

14. Verfahren (200) zur Bestimmung einer Konzentration eines Zielgases in einem Messgas, aufweisend die Verfahrensschritte (V1, V2, ...):
- (V1) Bereitstellen eines Flusses von Nullgas (K) durch eine erste Gaszuführung (21) zu einem Gassensor (10) in einem ersten Zeitabschnitt (t1),
- (V2) Bereitstellen eines Flusses von Messgas (M) durch eine zweite Gaszuführung (22) zu dem Gassensor (10) in einem zweiten Zeitabschnitt (t2), welcher von dem ersten Zeitabschnitt (t1) verschieden ist,
- (V3) Bereitstellen des Flusses von Nullgas (K) durch eine Gasabführung (23) während des zweiten Zeitabschnitts (t2), wobei die erste Gaszuführung (21) mit der Gasabführung (23) und diese mit einer Umgebung (U) der Vorrichtung (100) verbunden ist.
